# EUROPEAN PATENT APPLICATION

(11) **EP 1 394 243 A1**
(43) Date of publication of application: **03.03.2004**
(21) Application number: 03254693.9
(22) Date of filing: 28.07.2003
(51) Int. Cl.: C10M 135/16, C07D 233/48

(54) **Cyclic aminothioureas as additives for lubricating oils**

(30) Priority: 07.08.2002 US 401845 P
(71) Applicant: Rohm and Haas, Philadelphia, Pennsylvania 19106-2399 (US)
(72) Inventor: Mukkamala, Ravindranath, Landsdale Pennsylvania 19446 (US)
(74) Representative: Kent, Venetia Katherine

(57) **Abstract**

A composition comprising: (a) from 0.1% to 20% of at least one amino-substituted imidazolidinethione compound of formula I: 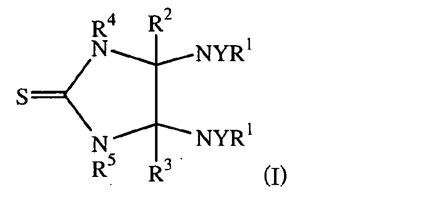 and (b) a lubricating oil.

## Description

### Background

This invention relates generally to cyclic aminothioureas useful as additives for lubricating oils.

Zinc dialkyldithiophosphates (ZDDP) are widely used as lubricant additives. The principal disadvantages of these compounds are that an ash residue is produced by the zinc as the additive is consumed, and that phosphorus is known to affect the efficiency of catalytic converters in motor vehicles, thereby causing emissions problems. Cyclic thiourea compounds useful as lubricant additives are disclosed in U.S. Patent No. 5,935,913. However, the compounds disclosed therein are not within the scope of the present invention.

The problem addressed by this invention is to find additional non-metallic, non-phosphorus-containing oil-soluble additives for lubricating oils.

### Statement of Invention

The present invention is directed to a composition comprising:
(a) from 0.1% to 20% of at least one amino-substituted imidazolidinethione compound of formula I: 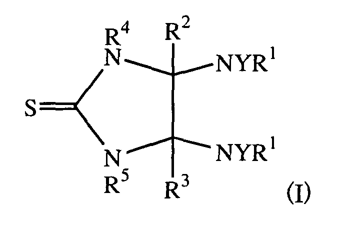 wherein R² and R³ independently are hydrogen, alkyl, alkenyl, aryl or aralkyl; or R² and R³ groups combine with carbon atoms of an imidazolidinethione ring to form a C₅-C₈ cycloalkyl or cycloalkenyl ring; R¹ is alkyl, alkenyl, aralkyl, or R¹ groups combine with nitrogen atoms to which they are attached and carbon atoms of an imidazolidinethione ring to form a five- to seven-membered heterocylic ring; R⁴ and R⁵ independently are hydrogen, alkyl, alkenyl, aryl, aralkyl, CHR⁶-CHR⁷-COOR⁸, -CR⁹R¹⁰NHR¹¹ or -C(Z)NHR¹²; R⁶ and R⁷ independently are hydrogen or C₁-C₄ alkyl; R⁸, R⁹ and R¹⁰ independently are hydrogen, alkyl, alkenyl, aryl or aralkyl; R¹¹ and R¹² independently are alkyl, alkenyl, aralkyl or aryl; Y is hydrogen, alkyl, alkenyl, aralkyl, -CHR⁶-CHR⁷-COOR⁸, -CR⁹R¹⁰NHR¹¹, -C(Z)NHR¹² or oxyl; and Z is O or S; and
(b) a lubricating oil.

The present invention is further directed to a method for improving the anti-wear characteristics of a lubricating oil by adding from 0.1% to 20% of a compound of formula I.

The present invention is further directed to a compound of formula (I), wherein R² and R³ independently are hydrogen, alkyl, alkenyl, aryl or aralkyl; or R² and R³ groups combine with carbon atoms of an imidazolidinethione ring to form a C₅-C₈ cycloalkyl or cycloalkenyl ring; R¹ is alkyl, alkenyl or aralkyl; R⁴ and R⁵ independently are hydrogen, alkyl, alkenyl, aryl, aralkyl, -CHR⁶-CHR⁷-COOR⁸, -CR⁹R¹⁰NHR¹¹ or -C(Z)NHR¹²; R⁶ and R⁷ independently are hydrogen or C₁-C₄ alkyl; R⁸, R⁹ and R¹⁰ independently are hydrogen, alkyl, alkenyl, aryl or aralkyl; R¹¹ and R¹² independently are alkyl, alkenyl, aralkyl or aryl; Y is hydrogen, alkyl, alkenyl, aralkyl, -CHR⁶-CHR⁷-COOR⁸, -CR⁹R¹⁰NHR¹¹, -C(Z)NHR¹² or oxyl; and Z is O or S.

The present invention is further directed to a lubricating oil composition containing the reaction product of a compound of formula (I) with an imine; an unsaturated carboxylic acid or ester; an isocyanate or isothiocyanate; or an alkyl, alkenyl or aralkyl group bearing a leaving group.

### Detailed Description

All percentages are weight percentages based on the entire composition described, unless specified otherwise. An "alkyl" group is a saturated hydrocarbyl group having from one to twenty-two carbon atoms in a linear, branched or cyclic arrangement, and having from 0 to 2 oxygen, nitrogen or sulfur atoms. Substitution on alkyl groups of one or more halo, hydroxy, alkoxy, alkanoyl or amido groups is permitted; alkoxy, alkanoyl and amido groups may in turn be substituted by one or more halo substituents. In one preferred embodiment, alkyl groups contain from one to twelve carbon atoms and from 0 to 1 oxygen, nitrogen or sulfur atoms; in another preferred embodiment, alkyl groups contain from 4 to 22 carbon atoms, and more preferably, no heteroatoms. An "alkenyl" group is an "alkyl" group in which at least one single bond has been replaced with a double bond. A "difunctional alkyl" or "difunctional alkenyl" group is an alkyl or alkenyl group having two points of attachment on the same or different carbon atoms, e.g., -CH₂-, -CH₂CH₂-, -CH(CH₂CH₃)-, and -CH=CH-. An "aryl" group is a substituent derived from an aromatic compound, including heterocyclic aromatic compounds having heteroatoms chosen from among nitrogen, oxygen and sulfur. An aryl group has a total of from five to twenty ring atoms, and has one or more rings which are separate or fused. Substitution on aryl groups of one or more halo, alkyl, alkenyl, hydroxy, alkoxy, alkanoyl or amido groups is permitted, with substitution by one or more halo groups being possible on alkyl, alkenyl, alkoxy, alkanoyl or amido groups. An "aralkyl" group is an "alkyl" group substituted by an "aryl" group. An "oxyl" substituent on a nitrogen atom is an -O· substituent, i.e., the nitrogen and the oxyl form a nitroxyl group. A "lubricating oil" is a natural or synthetic oil, or a mixture thereof, having suitable viscosity for use as a lubricant, e.g., as crankcase oil in an internal combustion engine, automatic transmission fluid, turbine lubricant, gear lubricant, compressor lubricant, metal-working lubricant, hydraulic fluid, etc.

In the composition comprising a compound of formula (I) in a lubricating oil, optionally, the R¹ groups in formula (I) combine with nitrogen atoms to which they are attached and carbon atoms of an imidazolidinethione ring to form a five- to seven-membered heterocylic ring. Preferably, the R¹ groups collectively represent a difunctional alkyl or alkylene group optionally containing a carbonyl or thiocarbonyl group. In a preferred embodiment where the two R¹ groups collectively represent the group -(CH₂)ₘ-C(X)-(CH₂)ₙ-, the following bicyclic system of formula (II) results: 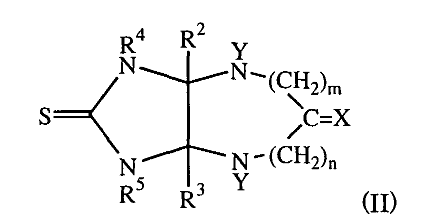 wherein m is 0, 1 or 2; n is 0, 1 or 2; provided that m+n≤2; and X is O or S. Preferably, R² and R³ independently are hydrogen, C₄-C₂₂ alkyl or C₄-C₂₂ alkenyl; most preferably, R² and R³ independently are C₄-C₂₂ alkyl. In a preferred embodiment where the R¹ groups collectively represent a thiocarbonyl group, i.e., m and n are zero and X is S, the following bicyclic system of formula (III) results: 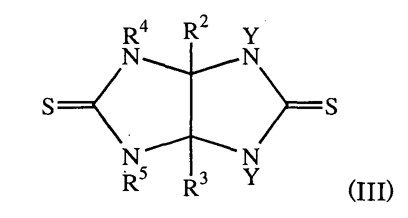 When Y, R⁴ and R⁵ in formula (III) are hydrogen, the symmetrical bicyclic structure represented by formula (IV) results.

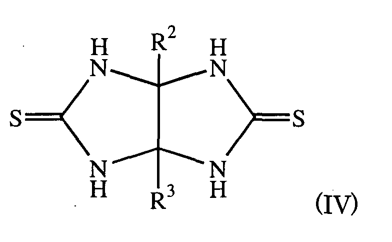

The present invention also is directed to a novel compound of formula (I), with the substituent definitions given herein for formula (I), except that the R¹ groups do not combine with other atoms to form a five- to seven-membered ring.

In another preferred embodiment of the invention, where R² and R³ groups in formula (I) combine with carbon atoms of an imidazolidinethione ring to form a C₅-C₈ cycloalkyl ring, the following bicyclic system of formula (V) results: 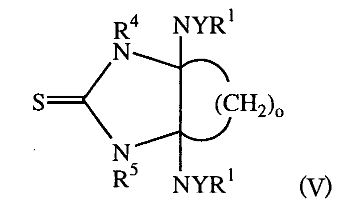 wherein o is 3, 4, 5 or 6.

In a preferred embodiment of the invention in which the R¹ groups join to form a five- to seven-membered heterocylic ring, the two R¹ groups collectively represent the group -(CH₂)ₘ-C(X)-(CH₂)ₙ-, and the R² and R³ groups join to form a C₅-C₈ cycloalkyl ring, the tricyclic system of formula (VI) results:

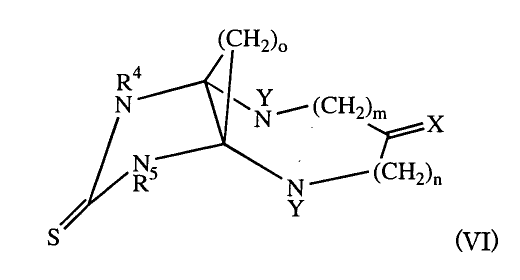

In one embodiment of the invention, a compound of formula (I) is prepared from thiourea and an acyclic glyoxal-based diimine, as shown below in Scheme 1: 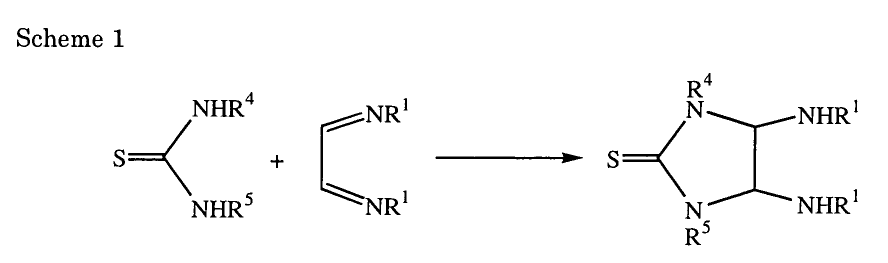 Preferably, R¹ is C₄-C₂₂ alkyl or C₄-C₂₂ alkenyl, more preferably C₆-C₂₂ alkyl, and most preferably C₁₀-C₂₂ alkyl. In one preferred embodiment, R¹ is derived from an unsubstituted C₁₆-C₂₂ alkyl amine, R¹NH₂, preferably one which is an oil-soluble amine. In one preferred embodiment, the alkyl amine is a tertiary alkyl primary amine., i.e., a primary amine in which the alkyl group is attached to the amino group through a tertiary carbon. Examples of commercially available tertiary alkyl primary amines are the Primene™ amines available from Rohm and Haas Company, Philadelphia, PA. Preferably, R⁴ and R⁵ independently are hydrogen or alkyl. Use of a cyclic diimine in which the R¹ groups combine to form a to form a five- to seven-membered heterocylic ring will result in a bicyclic product. When the two R¹ groups collectively represent the group -(CH₂)ₘ-C(X)-(CH₂)ₙ-, the product will have formula (II), as shown above.

In one embodiment, a compound of formula (I) is produced from an imine based on a diketone, as shown in Scheme 2: 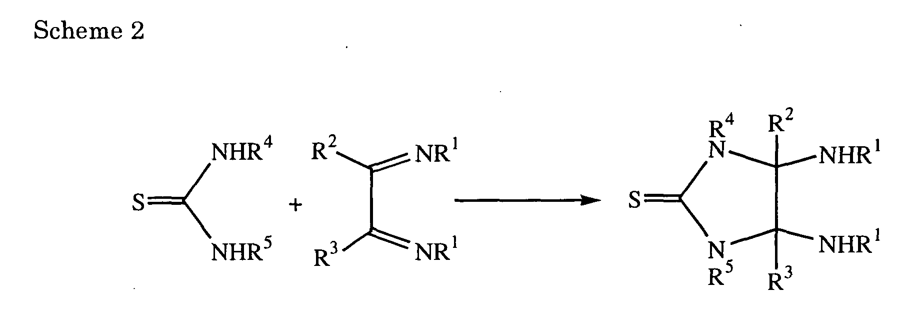 In Scheme 2, R² and R³ independently are alkyl, alkenyl, aryl, aralkyl or R² and R³ collectively are difunctional alkyl; preferably, R² and R³ are alkyl or collectively are difunctional alkyl. When the R² and R³ groups collectively represent a difunctional alkyl group, the product will have formula (V), as shown above.

Optionally, a compound produced as shown in Scheme 2, preferably one in which R¹ is a tertiary alkyl group, and R² and R³ are alkyl groups, is oxidized with hydrogen peroxide or a peracid to form a nitroxyl compound in which the - NHR¹ group has been converted into an -N(O·)R¹ group, as shown below i formula (VII).

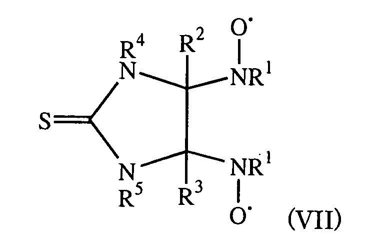

In one embodiment of the invention, a compound of formula (IV) is prepared from thiourea and a 1,2-dicarbonyl compound, as shown below in Scheme 3: 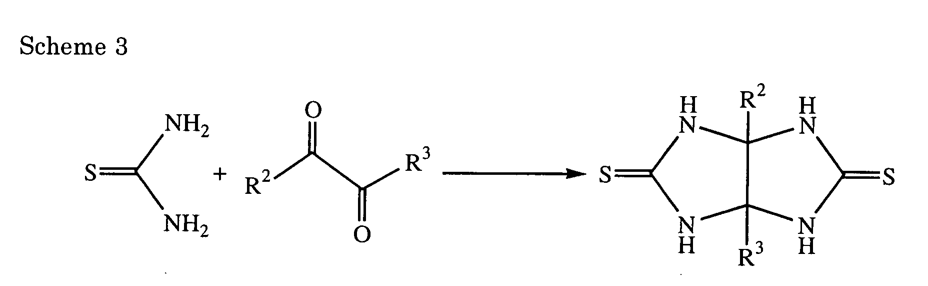 Preferably, R² and R³ are alkyl groups, most preferably C₁₀-C₂₂ alkyl groups. If a substituted thiourea is used in place of thiourea itself, a product of formula (III) results. In one preferred embodiment, a 1,2-dicarbonyl compound is used in which the R² and R³ groups are combined with the carbonyl carbon atoms of the 1,2-dicarbonyl compound to form a cyclic 1,2-dicarbonyl compound, resulting in a tricyclic product in which R² and R³ combine to form a third ring. In one preferred embodiment, R² and R³ combine to form a C₃-C₆ difunctional alkyl group, thereby resulting in a starting material which is a C₅-C₈ cyclic di-ketone, and a product containing a C₅-C₈ cycloalkyl ring.

In one aspect of the invention, a compound of formula (I) in which at least one of R⁴, R⁵ and Y is hydrogen, or at least one of R² and R³ contains a primary or secondary amine nitrogen atom, is allowed to react with a compound of formula CHR⁶=CR⁷COOR⁸ to produce a compound having a -CHR⁶CHR⁷COOR⁸ group on a thiourea nitrogen atom or an amine nitrogen atom. Preferably, R⁶ and R⁷ are hydrogen or methyl. Preferably, the compound of formula CHR⁶=CR⁷COOR⁸ is an alkyl or aralkyl acrylate having R⁶= R⁷=H and R⁸=alkyl or aralkyl; or a methacrylate ester having R⁶=H and R⁷=CH₃; or a crotonate ester having R⁷=H and R⁶=CH₃. In another aspect of this invention, a compound of formula (I) in which at least one of R⁴, R⁵ and Y is hydrogen, or at least one of R² and R³ contains a primary or secondary amine nitrogen atom, is alkylated with an imine, CR⁹R¹⁰=NR¹¹. Preferably, R¹¹ is alkyl, most preferably C₁₂-C₂₂ alkyl. Preferably, R⁹ and R¹⁰ independently are alkyl or hydrogen. In a preferred embodiment of the invention, CR⁹R¹⁰=NR¹¹ is a formaldehyde imine, CH₂=NR¹¹. In another aspect of this invention, a compound of formula (I) in which at least one of R⁴, R⁵ and Y is hydrogen, or at least one of R² and R³ contains a primary or secondary amine nitrogen atom, reacts with an isocyanate, R¹²NCO, or an isothiocyanate, R¹²NCS, to produce a compound having a -C(O)NHR¹² or a - C(S)NHR¹² group, respectively. Preferably, R¹² is aryl, alkyl or aralkyl, more preferably C₈-C₂₀ alkyl, aryl or aralkyl. A compound of formula (I) in which at least one of R⁴, R⁵ and Y is hydrogen, or at least one of R² and R³ contains a primary or secondary amine nitrogen atom, also may react with an alkyl, alkenyl or aralkyl group bearing a suitable leaving group, thereby introducing an alkyl, alkenyl or aralkyl group on a thiourea or amine nitrogen atom. Suitable leaving groups and conditions for such a reaction are well known to those skilled in the art; suitable leaving groups include, e.g., iodide, bromide, chloride, mesylate, tosylate and triflate.

As an example, the reaction of the compound of formula (IV) at one thiourea nitrogen with an imine, and with an acrylate is illustrated below in Scheme 4. Analogous products result from reaction of other compounds depicted herein with an imine or an acrylate at either a thiourea nitrogen atom or a primary or secondary amine nitrogen atom. 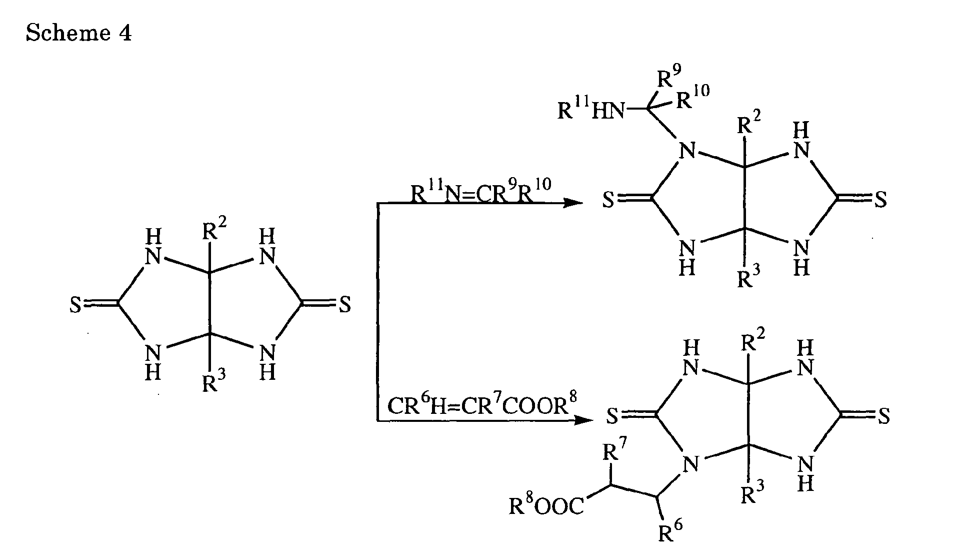 Substitution on more than one thiourea nitrogen in formula (IV), either from the same thiourea group or different thiourea groups, also is possible, depending on the stoichiometry and conditions of the reaction.

The composition of the present invention includes at least one compound of formula (I). In one embodiment of the invention, the composition contains at least two compounds of formula (I), and more preferably contains at least three compounds of formula (I). Preferably, the compound(s) of formula (I) is present in a lubricating oil in a total amount of at least 0.2%, more preferably at least 0.3%, and most preferably at least 0.4%. Preferably, the compound(s) of formula (I) is present in a lubricating oil in a total amount no greater than 10%, more preferably no greater than 5%, and most preferably no greater than 2%. Preferably, the compounds are soluble at the aforementioned levels.

Optionally, other additives typically used in lubricating oils are present in the composition. Such additives include, but are not limited to, other anti-wear additives, anti-corrosion additives, dispersants, detergents, antioxidants, antifoamants, friction modifiers, seal swell agents, demulsifiers, viscosity index improvers and pour point depressants. Other anti-wear additives that can be used in combination with the compound of formula (I) include the commercial products known as ZDDP, which are zinc dialkyldithiophosphates. In addition to improving the anti-wear characteristics of lubricating oils, the compound of formula (I) typically also improves anti-corrosion characteristics and functions as an anti-oxidant.

## Claims

1. A composition comprising:
(a) from 0.1% to 20% of at least one amino-substituted imidazolidinethione compound of formula I: 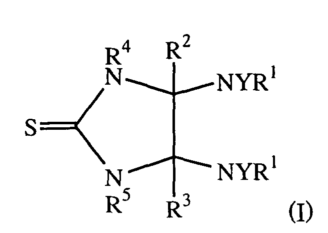 wherein R² and R³ independently are hydrogen, alkyl, alkenyl, aryl or aralkyl; or R² and R³ groups combine with carbon atoms of an imidazolidinethione ring to form a C₅-C₈ cycloalkyl or cycloalkenyl ring; R¹ is alkyl, alkenyl, aralkyl, or R¹ groups combine with nitrogen atoms to which they are attached and carbon atoms of an imidazolidinethione ring to form a five- to seven-membered heterocylic ring; R⁴ and R⁵ independently are hydrogen, alkyl, alkenyl, aryl, aralkyl, -CHR⁶-CHR⁷-COOR⁸, -CR⁹R¹⁰NHR¹¹ or -C(Z)NHR¹²; R⁶ and R⁷ independently are hydrogen or C₁-C₄ alkyl; R⁸, R⁹ and R¹⁰ independently are hydrogen, alkyl, alkenyl, aryl or aralkyl; R¹¹ and R¹² independently are alkyl, alkenyl, aralkyl or aryl; Y is hydrogen, alkyl, alkenyl, aralkyl, -CHR⁶-CHR⁷-COOR⁸, -CR⁹R¹⁰NHR¹¹, -C(Z)NHR¹² or oxyl; and Z is O or S; and
(b) a lubricating oil.

2. The composition of claim 1 in which R¹ is alkyl, alkenyl, aralkyl or R¹ groups collectively are -(CH₂)ₘ-C(X)-(CH₂)ₙ-, wherein m is 0, 1 or 2; n is 0, 1 or 2; provided that m+n≤2; and X is O or S.

3. The composition of claim 2 in which R¹ groups collectively are -(CH₂)ₘ-C(X)-(CH₂)ₙ-, m=n=0 and X is S, so that the R¹ groups collectively represent thiocarbonyl; R² and R³ independently are C₄-C₂₂ alkyl groups; and R⁴ and R⁵ independently are hydrogen, alkyl, alkenyl, aryl or aralkyl.

4. The composition of claim 1 in which R¹ is C₄-C₂₂ alkyl or C₄-C₂₂ alkenyl.

5. The composition of claim 4 in which at least one of R⁴, R⁵ and Y is - CHR⁶-CHR⁷- COOR⁸, -CR⁹R¹⁰NHR¹¹ or - C(Z)NHR¹².

6. The composition of claim 4 in which R¹ is tertiary alkyl, R² and R³ are alkyl, and Y is oxyl.

7. A compound of formula I: 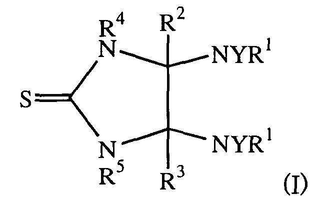 wherein R² and R³ independently are hydrogen, alkyl, alkenyl, aryl or aralkyl; or R² and R³ groups combine with carbon atoms of an imidazolidinethione ring to form a C₅-C₈ cycloalkyl or cycloalkenyl ring; R¹ is alkyl, alkenyl or aralkyl; R⁴ and R⁵ independently are hydrogen, alkyl, alkenyl, aryl, aralkyl, -CHR⁶-CHR⁷-COOR⁸, -CR⁹R¹⁰NHR¹¹ or -C(Z)NHR¹²; R⁶ and R⁷ independently are hydrogen or C₁-C₄ alkyl; R⁸, R⁹ and R¹⁰ independently are hydrogen, alkyl, alkenyl, aryl or aralkyl; R¹¹ and R¹² independently are alkyl, alkenyl, aralkyl or aryl; Y is hydrogen, alkyl, alkenyl, aralkyl, -CHR⁶-CHR⁷-COOR⁸, -CR⁹R¹⁰NHR¹¹, -C(Z)NHR¹² or oxyl; and Z is O or S.

8. The compound of claim 7 in which R¹ is C₄-C₂₂ alkyl or C₄-C₂₂ alkenyl.

9. The compound of claim 8 in which at least one of R⁴, R⁵ and Y is - CHR⁶-CHR⁷- COOR⁸, -CR⁹R¹⁰NHR¹¹ or -C(Z)NHR¹².

10. The compound of claim 8 in which R¹ is tertiary alkyl, R² and R³ are alkyl, and Y is oxyl.
